# EUROPEAN PATENT APPLICATION

(11) **EP 1 870 110 A1**
(43) Date of publication of application: **26.12.2007**
(21) Application number: 06729793.7
(22) Date of filing: 23.03.2006
(51) Int. Cl.: A61K 48/00, A61K 31/7088, A61K 9/14, A61K 38/17, A61K 47/12, A61K 47/18, A61P 17/00

(54) **PREPARATION COMPRISING MICROPARTICLES OF COMPLEX COMPOSED OF NUCLEIC ACID MOLECULE AND COLLAGEN**

(30) Priority: 24.03.2005 JP 2005086318
(71) Applicant: Dainippon Sumitomo Pharma Co., Ltd., Osaka 541-8524 (JP); KOKEN CO., LTD., Toshima-ku, Tokyo 171-0031 (JP)
(72) Inventor: MAEDA, Miho, c/o Dainippon Sumitomo Pharma Co.,Ltd., Ibaraki-shi, Osaka 5670878 (JP); KISHIMOTO, Yoshiko, Ogaki-shi, Gifu 5030835 (JP)
(74) Representative: Vossius & Partner
(86) International application number: PCT/JP2006/305845
(87) International publication number: WO 2006/101173

(57) **Abstract**

The present invention relates an additive comprising at least one substance selected from arginine, trometamol, meglumine, lysine, histidine, monoethanolamine, diethanolamine, triethanolamine, succinic acid, citric acid, tartaric acid, lactic acid, and salts thereof, which is useful in the prevention of aggregation of fine particulate complexes of a nucleic acid and collagen, and the production of a preparation comprising particulate complexes of controlled size which is suited for transporting a nucleic acid into cells.

## Description

### TECHNICAL FIELD

The present invention relates to a preparation comprising fine particulate complexes of controlled size of a nucleic acid molecule and collagen, and particularly to a fine particulate complex of a nucleic acid molecule and collagen formed by adding a certain additive, a preparation which comprises the fine particulate complex and is useful for transferring the nucleic acid molecule into cells, a process for preparing the same, and the like.

### BACKGROUND ART

Collagen, which shows good biocompatibility and has heretofore been used in implantable medical devices, has a wide range of potential of applications including regenerative medicines, reagents or kits in the biological technology, and the like. Further, the effects of collagen were shown that collagen stabilized nucleic acid molecules by forming complexes of the nucleic acid molecules and collagen therewith through the electrostatic interaction and facilitated the nucleic acid molecules transfer into tissues, and the DDS using collagen was considered to be a prospective DDS of the nucleic acid molecules (Patent documents 1, 2 and 3). In particular, atelocollagen, which lacks antigenic portions and is soluble, has a high application value as a medicinal substance.

In the gene therapy, wherein diseases are treated by supplementing with normal genes in cells and by repairing or modifying genetic defects and the like, a nucleic acid molecule encoding an objective enzyme or cytokine is transferred into cells of a patient so that an intended substance is produced from the nucleic acid molecule in vivo to treat the disease. However, gene transfer efficiency to target cells is low when a gene is used by itself. To increase gene transfer efficiency and to improve therapeutic effectiveness, a method which uses virus such as adenovirus as vectors (Non-patent documents 1-6 and the cited reference thereof) or liposome formulations (Non-patent documents 7-9), or the like, has been employed. In addition, gene carriers such as basic lipids (Non-patent document 10 and many lipids developed thereafter) and basic-polymer-based compounds (typical examples: polyethyleneimine (Non-patent document 11) and the like) have been developed. These vectors are applicable to nucleic acid formulations, wherein the nucleic acid includes not only genes used in the gene therapy but also antisense DNAs, siRNA and the like which regulate intracellular gene expression.

Further, a method which uses a complex of a nucleic acid molecule with collagen, a biopolymer, to increase the gene transfer efficiency and improve therapeutic effectiveness has been proposed (Patent document 3). Because of good biocompatibility, collagen is expected to be applicable to pharmaceuticals.

From the standpoint of stability of nucleic acid molecules or application to pharmaceuticals, it is preferred to prepare and maintain complexes in a condition of neutral pH, which, however, is rather problematic from the standpoint that collagens characteristically tend to assemble to form precipitates in an aqueous solution of around neutral pH. In particular, the facts that collagens assemble regularly to form fibrils under physiological conditions or that agglutination is accelerated by formation of complexes of nucleic acid molecules can impair the processing features or be an obstacle in the manufacture of pharmaceuticals showing consistent quality. Although the formation of huge insoluble aggregates of complexes between collagen and a nucleic acid molecule may be advantageously prolong the release from sustained-release preparations of depot-type, it is not preferred when an extensive in vivo distribution or efficient transfer into cells are required. For the efficient incorporation into cells, the size of complex is preferably not larger than several µm, and huge aggregates are undesirable. It is said that particles of complexes are preferably not greater than 5 µm in size in order to achieve an extensive in vivo distribution through the blood flow in view of the diameter (5 - 10 µm) of capillary vessels. Accordingly, collagen in a complex with a nucleic acid molecule is preferably in the form of a single molecule or a fine fibril composed of a several to several tens collagen molecules.

Fibrillary collagens tend to be solubilized in the presence of 0.9 M - 1.0M NaCl at neutral pH, which becomes more effective by adding glucose or sucrose (0.25 - 0.5M) having inhibitory activity on the formation of collagen fibrils at neutral pH (with a salt added) according to "Collagen Jikken-ho", Kodan-sha, page 3. Further, as a fiber disassembly agent, which disassembles fibrillar collagens into non-fibrillar ones, biocompatible alcohols, amino acids and the like are described (Patent document 4). However, a composition disclosed in the Patent document 4 does not contain a nucleic acid molecule and the document is unrelated to a complex composed collagen and a nucleic acid molecule.

Collagen by itself can be dissolved homogenously and clearly but immediately forms complexes of several µm or longer on addition of nucleic acid molecules, and then the complexes form aggregate which tends to be greatly accelerated by warming to a temperature around room temperature. As a result, aggregated complexes of 10 µm or larger are easily formed to give a clouded suspension, followed by precipitation. Further, aggregation is accelerated by preservation and freeze-thawing procedures.

The present inventors have found for the first time that the above-mentioned methods (solubilization or use of fiber disassembly agent) conventionally used for preventing aggregation of collagens may be useful when solely collagens are involved, but are useless to prevent the aggregation of collagens or the formation of huge particles when nucleic acid molecules are participated therein. Accordingly, the development of fine particulate preparation comprising a complex essentially composed of collagen and nucleic acid molecule, which is capable of maintaining uniform dispersibility stably, in other words, which is uniform and shows splendid dispersibility and is suited for practical use is strongly demanded.

| | |
|---|---|
| Patent document 1: | JP-A-H9-71542 |
| Patent document 2: | WO2001/97857 |
| Patent document 3: | WO2003/297 |
| Patent document 4: | JP-A-H9-99052 |
| Non-patent document 1: | Cardiovascular Research, 28, 445 (1994) |
| Non-patent document 2: | Science, 256, 808 (1992) |
| Non-patent document 3: | Gastroenterology, 106, 1076 (1994) |
| Non-patent document 4: | TIBTECH, 11, 182 (1993) |
| Non-patent document 5: | J. Biol. Chem, 266, 3361 (1991) |
| Non-patent document 6: | Nature Medicine, 1, 583 (1995) |
| Non-patent document 7: | Biochem Biophys Acta, 1097, 1 (1991) |
| Non-patent document 8: | Human Gene Therapy, 3, 399 (1992) |
| Non-patent document 9: | Proc. Natl. Acad. Sci.USA, 89, 11277 (1992) |
| Non-patent document 10: | Proc. Natl. Acad. Sci.USA, 84, 7413 (1987) |
| Non-patent document 11: | Proc. Natl. Acad. Sci.USA, 92, 7297 (1995) |

### DISCLOSURE OF THE INVENTION

A purpose of the present invention is to provide a fine particulate preparation comprising a nucleic acid molecule, which can maintain the uniform dispersibility stably, in other word, which shows uniform splendid dispersibility and is suitable for practical use.

In particular, the present invention is related to a preparation comprising three substances as essential components i.e., nucleic acid molecule/ collagen/ additive, which comprises a complex of controlled size and whereby solved technical problems which could not be solved by simply applying a fiber disassembly agent of collagen to a complex of nucleic acid molecule and collagen.

The present invention is based on a fine particle of controlled size comprising a complex of a nucleic acid molecule and collagen. Specifically, the present invention provides a fine particulate complex formed by adding a certain kind of additive to a nucleic acid molecule and collagen, and a preparation for transferring (transporting) a nucleic acid molecule into cells which comprises said fine particulate complex. The present invention also provides a process for preparing the fine particulate complex, or the preparation. Controlling the particle size of complexes is inevitable from the viewpoints of not only possible influences on in vivo kinetics or effects but also quality control particularly in the application to pharmaceuticals.

The present inventors have studied intensively in the consideration of above-mentioned problems, and found that a certain kind of the organic base or acid, when coexisted with complexes of collagen and a nucleic acid molecule, can prevent the formation of complexes having a size of 10µm or greater while maintaining the affinity of these two substances. It was unexpected finding that aggregation can be prevented even when collagen forms a complex with a nucleic acid.

The present invention is related the followings.
1. An additive for making a complex of a nucleic acid molecule and collagen in the form of a fine particle of controlled size, which comprises at least one substance selected from arginine, trometamol, meglumine, lysine, histidine, monoethanolamine, diethanolamine, triethanolamine, succinic acid, citric acid, tartaric acid, lactic acid, and salts thereof.
2. An aqueous solution comprising an additive described in 1 above and collagen, which is for making a complex of a nucleic acid molecule and collagen in the form of fine particle of controlled size; or
   an aqueous solution comprising an additive described in 1 above and collagen, which is for preparing a fine particulate complex of controlled size comprising a nucleic acid molecule and collagen by adding the nucleic acid to the aqueous solution.
3. The aqueous solution according to 2 above, wherein
   (1) the total concentration of additive is 1 - 20%,
   (2) the concentration of collagen is 0.01% - 2%, and
   (3) the additive is selected from arginine, trometamol, meglumine, lysine, monoethanolamine, triethanolamine, citric acid, tartaric acid, and salts thereof.
4. The aqueous solution according to 2 above, wherein the additive is selected from arginine, trometamol, meglumine, lysine, and salts thereof.
   4-1. The aqueous solution according to 2 above, wherein the additive is selected from arginine, lysine, and salts thereof.
   4-2. An aqueous solution comprising an additive described in 1 above for reconstituting a dry formulation comprising a nucleic acid molecule and collagen into a liquid formulation, which is for making a complex of a nucleic acid molecule and collagen in the form of a fine particle of controlled size.
5. The aqueous solution according to 4 above, wherein
   (1) the total concentration of additive is 1 - 10%, and/or
   (2) the pH of the aqueous solution is 5 - 9, and
   (3) the additive is selected from arginine, trometamol, meglumine, lysine, monoethanolamine, triethanolamine, citric acid, tartaric acid, and salts thereof; preferably the additive is selected from arginine, trometamol, meglumine, lysine, and salts thereof; and more preferably the additive is selected from arginine, lysine, and salts thereof.
6. A preparation comprising an additive described in 1 above, a nucleic acid molecule and collagen, wherein the nucleic acid molecule is complexed with collagen to form fine particles of controlled size.
7. The preparation according to 6 above, wherein
   (1) the concentration of nucleic acid molecule is 0.001 - 100 mg/ml, preferably 0.001 - 50 mg/ml, more preferably 0.001 - 10 mg/ml,
   (2) the concentration of collagen is 0.01 % - 2 %,
   (3) the total concentration of additives is 1 - 10 %, and/or
   (4) the pH of aqueous solution is 6 - 8.
8. The preparation according to 6 above, wherein the fine particles of controlled size are complexes which have a controlled size enabling the complexes to maintain the dispersed state while preventing rapid sedimentation, and which can be administered through an ordinary injection needle without difficulty.
9. The preparation according to 8 above, wherein the fine particles of controlled size are complexes which have a size adapted to pass through a 23 to 21 gauge (inside diameter ("ID"): about 0.4 - 0.6 mm) injection needle used for intra-venous or -arterial administration or intramuscular administration, more preferably a 27 to 24 gauge (ID: about 0.2 - 0.4 mm) injection needle used for intra- or sub-cutaneous administration.
10. The preparation according to 6 above, wherein the fine particles of controlled size are particles of not greater than 100 µm preferably not greater than 10 µm.
11. The preparation according to 6 above, wherein at least 70 % of fine particles of controlled size are particles of not greater than 10 µm.
12. The preparation according to 6 above, wherein at least 80 % of fine particles of controlled size are particles of not greater than 10 µm.
13. The preparation according to 6 above, wherein at least 70 % of fine particles of controlled size are particles of not greater than 5 µm.
14. The preparation according to 6 above, wherein at least 80 % of fine particles of controlled size are particles of not greater than 5 µm.
15. A lyophilized formulation obtained by freeze-drying the solution described in 7 above.
16. An aggregation inhibitor for fine particulate complexes of a nucleic acid molecule and collagen, which comprises at least one substance selected from arginine, trometamol, meglumine, lysine, histidine, monoethanolamine, diethanolamine, triethanolamine, and salts thereof.
17. An additive comprising at least one substance selected from arginine, trometamol, meglumine, lysine, triethanolamine, and salts thereof, which is:
   (1) for making a complex of a nucleic acid molecule and collagen in the form of a fine particle of controlled size,
   (2) for preventing fine particles from aggregating and allowing fine particles to well disperse in a liquid formulation which comprises fine particulate complexes of controlled size comprising a nucleic acid molecule and collagen, and/or
   (3) for maintaining the prevention of aggregation of fine particles and the good dispersibility of the particles in a liquid formulation reconstituted from a dry formulation, which liquid formulation comprises fine particulate complexes of controlled size comprising a nucleic acid molecule and collagen.
18. An aqueous solution containing the additive described in 17 above and collagen, which is for making a complex of a nucleic acid molecule and collagen in the form of a fine particle of controlled size.
19. The aqueous solution according to 18 above, wherein
   (1) the total concentration of additive is 1 - 20%, and
   (2) the concentration of collagen is 0.01% - 2%.
20. An aqueous solution comprising an additive described in 17 above for reconstituting a dry formulation comprising a nucleic acid molecule and collagen into a liquid formulation, which is:
   (1) for making a complex of a nucleic acid molecule and collagen in the form of a fine particle of controlled size, and/or
   (2) for preventing fine particles from aggregating and allowing the fine particles to well disperse in a reconstituted liquid formulation which comprises fine particlulate complexes of controlled size comprising a nucleic acid molecule and collagen.
21. The aqueous solution according to 20 above, wherein
   (1) the total concentration of additive is 1 - 10%, and
   (2) the pH of the aqueous solution is 5 - 9.
22. A preparation comprising an additive described in 17 above, a nucleic acid molecule and collagen, wherein the nucleic acid molecule is complexed with collagen to form a fine particle of controlled size.
23. The liquid formulation according to 18 above, wherein
   (1) the concentration of nucleic acid molecule is 0.001 - 100 mg/ml, preferably 0.001 - 50 mg/ml, more preferably 0.001 - 10 mg/ml,
   (2) the concentration of collagen is 0.001 % - 10 %, preferably 0.01 % - 2 %, more preferably 0.01 % - 0.5 %,
   (3) the total concentration of additive is 1 - 10 %, and/or
   (4) the pH of aqueous solution is 5- 9, more preferably 6 - 8.
24. The preparation according to 6 above, wherein the fine particles of controlled size are complexes which have a size enabling the complexes to maintain the dispersed state while preventing rapid sedimentation, and which can be administered through an ordinary injection needle without difficulty.
25. The preparation according to 8 above, wherein the fine particles of controlled size are complexes which have a size adapted to pass through a 23 to 21 gauge (ID: about 0.4 - 0.6 mm) injection needle used for intra-venous or -arterial administration or intramuscular administration, more preferably a 27 to 24 gauge (ID: about 0.2 - 0.4 mm)injection needle used for intra- or sub-cutaneous administration.
26. The preparation according to 8 above, wherein the fine particles of controlled size are particles of not greater than 100 µm preferably not greater than 10 µm.
27. The preparation according to 8 above, wherein at least 70 % of fine particulate complexes of controlled size are not greater than 10 µm in size.
28. The preparation according to 8 above, wherein at least 80 % of fine particulate complexes of controlled size are not greater than 10 µm in size.
29. The preparation according to 8 above, wherein at least 70 % of complexes of collagen and a nucleic acid molecule in fine particulate complexes of controlled size are not greater than 5 µm in size.
30. The preparation according to 8 above, wherein at least 80 % of complexes of collagen and a nucleic acid molecule in the fine particulate complexes of controlled size are not greater than 5 µm in size.
31. The preparation according to 7 above, wherein the fine particulate complexes of a nucleic acid molecule and collagen of controlled size are prevented from aggregating and well dispersed without aggregation under non-refrigeration conditions around a temperature ranging from room temperature to body temperature for at least 12 hours.
32. An agent for transferring a nucleic acid molecule into desired cells, which comprises the preparation described in 31 above as an active ingredient.
33. The aqueous solution according to 4 above wherein the collagen is atelocollagen.
34. The preparation according to 7 above wherein the collagen is atelocollagen.
35. The preparation according to 7 above wherein the nucleic acid molecule is selected from (1) to (6) below:
   (1) a DNA oligonucleotide,
   (2) a plasmid DNA,
   (3) an RNA oligonucleotide,
   (4) an RNA/DNA chimera oligonucleotide,
   (5) an antisense DNA, and
   (6) a siRNA.
36. The preparation according to 35 above wherein the nucleic acid molecule is an antisense DNA.
37. A soft coating agent for a complex of a nucleic acid molecule and collagen comprising as an active ingredient at least one substance selected from arginine, trometamol, meglumine, lysine, histidine, monoethanolamine, diethanolamine, triethanolamine, succinic acid, citric acid, tartaric acid, lactic acid, and salts thereof.
38. A soft coating agent for a complex of a nucleic acid molecule and collagen comprising as an active ingredient at least one substance selected from arginine, trometamol, meglumine, lysine, and salts thereof.
39. An aggregation inhibitor for a complex of a nucleic acid molecule and collagen comprising as an active ingredient the soft coating agent described in 37 or 38 above.
40. A process of preparing a preparation comprising fine particulate complexes of a nucleic acid molecule and collagen having an average particle size of 100 µm or below, which comprises subjecting a nucleic acid molecule and collagen to complexation in the presence of an additive described in 1 above at pH 5 - 9.

### BRIEF DESCRIPTION OF DRAWINGS

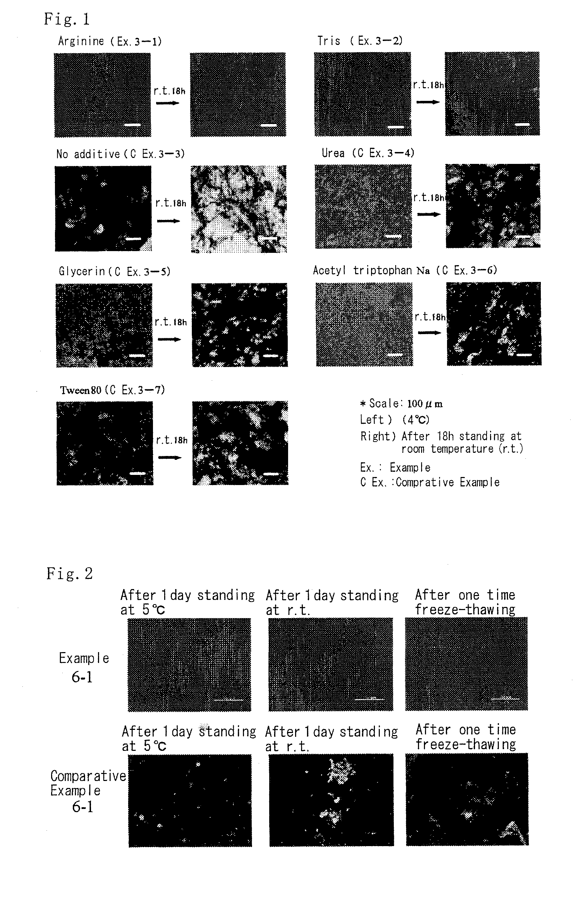

Figure 1 is a micrograph in substitution for a drawing which shows inhibitory effect of a various kinds of additives on the aggregation of fine particulate complexes. In the Comparative Example 3-5 where glycerin (glycerol) disclosed as a typical "fiber disassembly agent of collagen" in the Patent document 4 was used, aggregates (>10 µm) were observed after mixing collagen with DNA, which increased in size while standing at room temperature, indicating that glycerin does not have inhibitory effect on the aggregation of complexes of collagen and DNA.

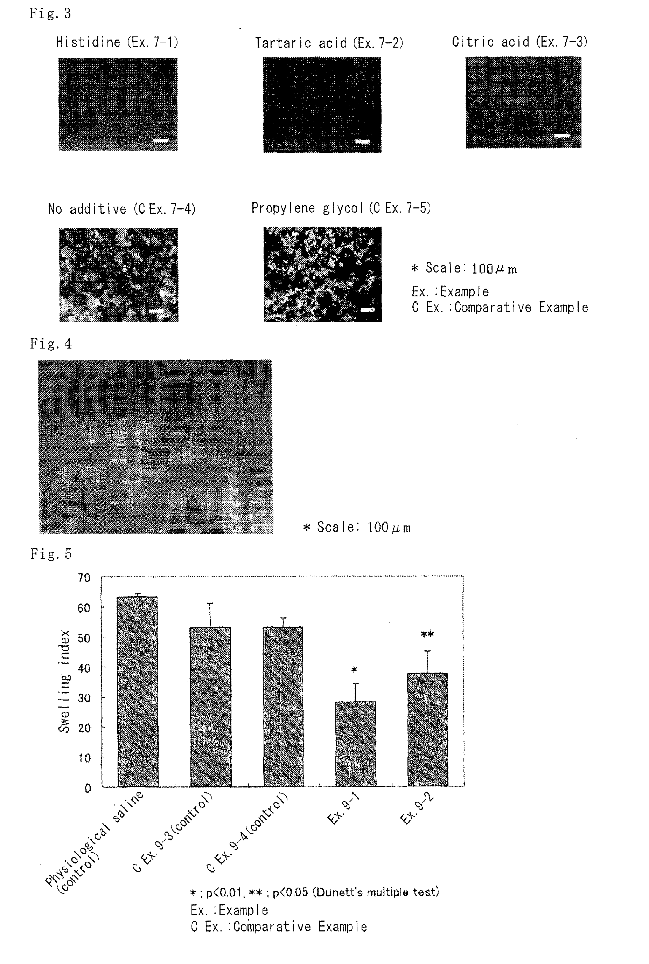

Figure 2 is a micrograph in substitution for a drawing which shows inhibitory effect of arginine on the aggregation of complexes due to microparticulation, temperature, and freeze-thawing treatments.
Figure 3 is a micrograph in substitution for a drawing which shows effect of a various kinds of additives on the microparticulation of complexes. In the Comparative Examples 7-5 wherein propylene glycol disclosed as a typical "fiber disassembly agent of collagen" in the Patent document 4 was used, many huge aggregates (>100 µm) were observed when collagen and DNA were mixed, indicating that propylene glycol does not have the microparticulation effect on the complexes of collagen and DNA.
Figure 4 is a micrograph in substitution for a drawing showing inhibitory effect of a preparation of the present invention on the aggregation of complexes in a preparation containing a high concentration (40mg/ml) of nucleic acid molecule. Scale: 100 m
Figure 5 is a bar graph showing the degree of ear swelling in mouse models of dermatitis 24 hours after sensitization and administration of a preparation. The preparation of the present invention comprising antisense ICAM-1, as a preparation of fine particulate complex, showed higher antiinflammatory effect (ear-swelling-inhibitory effect) compared to single administration of antisense ICAM-1.

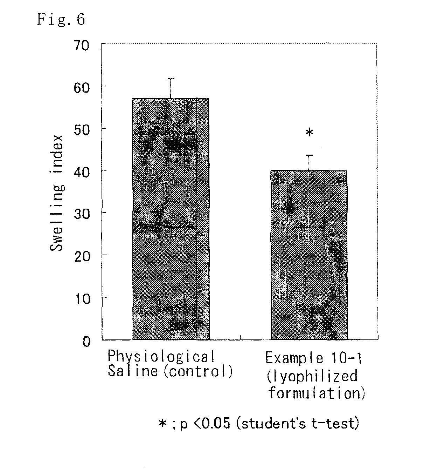

Figure 6 is a bar graph showing the degree of ear swelling in mouse models of dermatitis 24 hours after sensitization and administration of a preparation. The lyophilized formulation of the present invention comprising antisense ICAM-1 showed a significant antiinflammatory effect (ear-swelling-inhibitory effect), demonstrating that the fine particulate complex of the present invention is useful also as a lyophilized formulation.

### BEST MODE FOR CARRYING OUT THE INVENTION

### (1) Additives

The preparation/formulation of the present invention is characterized in that it comprises a particular additive, a nucleic acid molecule and collagen and that the nucleic acid molecule is complexed with collagen to form fine particles of controlled size.

The present inventors intensively studied and found that arginine, trometamol, meglumine, lysine, histidine, monoethanolamine, diethanolamine, triethanolamine, succinic acid, citric acid, tartaric acid, lactic acid, and salts thereof are able to not only prevent aggregation of collagens and/or complexes of a nucleic acid molecule and collagen, but also make a complex of a nucleic acid molcule and collagen in the form of a fine particle of controlled size, without inhibiting the complexation of collagen with a nucleic acid molecule.

From this aspect, as the first embodiment, the present invention provides an additive for making a complex of a nucleic acid molecule and collagen in the form of a fine particle of controlled size, which comprises at least one substance selected from arginine, trometamol, meglumine, lysine, histidine, monoethanolamine, diethanolamine, triethanolamine, succinic acid, citric acid, tartaric acid, lactic acid, and salts thereof.

The present inventors have further found that organic bases, i.e., arginine, trometamol, meglumine, lysine, histidine, monoethanolamine, diethanolamine, triethanolamine, and salts thereof, are able to not only make a complex of a nucleic acid molecule and collagen in the form of a fine particle of controlled size, but also prevent the fine particle from aggregating and allow the fine particle to well disperse in a liquid formulation comprising fine particulated complex of a nucleic acid molecule and collagen of controlled size, and/or maintain the prevention of aggregation of fine particle and the good dispersibility of the same even in a liquid formulation reconstituted from a dry formulation, which liquid formulation comprises fine particulate complexes of a nucleic acid molecule and collagen having a controlled size.

From this aspect, the present invention provides an additive comprising at least one substance selected from arginine, trometamol, meglumine, lysine, triethanolamine, and salts thereof, which is:
(1) for making a complex of a nucleic acid molecule and collagen in the form of a fine particle of controlled size,
(2) for preventing fine particles from aggregating and allowing fine particles to well disperse in a liquid formulation which comprises fine particulate complexes of controlled size of a nucleic acid molecule and collagen, and/or
(3) for maintaining the prevention of aggregation of fine particles and the good dispersibility of the particles even in a liquid formulation reconstituted from a dry formulation, which liquid formulation comprises fine particulate complexes of controlled size of a nucleic acid molecule and collagen.

As used herein, "salts" include salts of the above-mentioned organic bases with hydrochloric acid, phosphoric acid or an organic acid selected from citric acid, tartaric acid, succinic acid, lactic acid and acetic acid, and salts of these acids with a base selected from sodium hydroxide, arginine, trometamol, meglumine, lysine, histidine, monoethanolamine, diethanolamine and triethanolamine.

Without being limited to a particular theory, it is believed that the additive of the present invention effectively prevents aggregation while maintaining the affinity of collagen and a nucleic acid molecule through the ion-coating, namely, soft-coating, around collagen or a complex. Such an effect of soft coating with ion has not been recognized in regard to a salt such as NaCl, sodium phosphate, or glucose which is commonly used as a fiber disassembly agent of collagen.

The additive of the present invention may further contain another ingredient(s) for the purpose of stabilization, pH adjustment, isotonization, and the like. Organic bases among additives of the present invention can be used as a pH adjustor, in combination with hydrochloric acid, phosphoric acid, an organic acid such as citric acid, tartaric acid, succinic acid, lactic acid, acetic acid, or the like.

As one of effects, the present invention makes it possible to preserve a preparation in a frozen state or to convert the same into a lyophilized (freeze-dried) formulation. Cryopreservation is necessary to maintain a solution comprising a complex of a nucleic acid molecule and collagen stably for a long period of time. In the absence of the present invention, cryopreserved formulation, when thawed, often causes aggregation and production of huge particles. Examples of other method of long-term preservation include lyophilization to give a lyophilized formulation. The production of lyophilized formulations comprises the freezing step followed by drying step. Lyophilized formulations are generally dissolved by adding a solvent at the time of use, but aggregation is apt to occur during the series of processes which often impairs the solubility. According to the present invention, aggregation is prevented and fine particles disperse well to the same extent as before lyophilization. Further, an excipient and a solubilizing agent can be added as an additive other than a stabilizer, a pH adjustor and an isotonizing agent, and saccharides are usable.

### (2) Aqueous Solution Comprising Additive and Collagen

The preparation of the present invention comprising a particular additive, a nucleic acid molecule and collagen, which is characterized by that the nucleic acid molecule is complexed with collagen to form a fine particle of controlled size, can be prepared using an aqueous solution comprising an additives of the present invention and collagen.

In this embodiment, the present invention provides an aqueous solution comprising an additive of the present invention and collagen for making a complex of nucleic acid molecule and collagen in the form of a fine particle of controlled size.

The aqueous solution can be used for dissolving lyophilized nucleic acid product or for diluting an aqueous solution of nucleic acid. The aqueous solution of the present invention, when applied to such a nucleic acid molecule, allows the nucleic acid molecule and collagen contained therein to form a fine particulate complex of controlled size. Thus, it becomes possible to obtain a fine particulate complex of controlled size comprising a nucleic acid and collagen by using the aqueous solution of the present invention comprising an additive and collagen, specifically, by just adding the aqueous solution to a nucleic acid having been synthesized or isolated in respective facilities or one offered commercially as an aqueous solution or a lyophilized product, or in the form of a plate on which a nucleic acid is applied.

The aqueous solution of this embodiment is preferably an aqueous solution wherein,
(1) the total concentration of additive is 1 - 20%, and
(2) the concentration of collagen is 0.01% - 2%.

More preferably, the additive is selected from arginine, trometamol, meglumine, lysine, monoethanolamine, triethanolamine, citric acid, tartaric acid, and salts thereof, and still more preferably, the additive is selected from arginine, trometamol, meglumine, lysine, and salts thereof, and most preferably, the additive is selected from arginine, lysine, and salts thereof.

### (3) Aqueous Solution for Reconstituting Dry formulation to Liquid Formulation

Pharmaceuticals for injection are generally provided as lyophilized products in consideration of stability of active ingredients, which are converted into a liquid formulation using distilled water for injection at the time of use. The present inventors have found that if the additive etc. of the present invention is included in a solution for reconstituting a dry formulation to a liquid formulation, it is possible to make a complex of a nucleic acid and collagen in the form of a desired fine particle.

In this embodiment, the present invention provides an aqueous solution comprising an additive of the present invention for reconstituting a dry formulation comprising a nucleic acid molecule and collagen into a liquid formulation, which solution is for making a complex of a nucleic acid molecule and collagen in the form of a fine particle of controlled size.

The aqueous solution of this embodiment is preferably an aqueous solution wherein,
(1) the total concentration of additive is 1 - 10%, and/or
(2) the pH of the aqueous solution is 5 - 9.

More preferably, the additive is selected from arginine, trometamol, meglumine, lysine, monoethanolamine, triethanolamine, citric acid, tartaric acid, and salts thereof, and still more preferably, the additive is selected from arginine, trometamol, meglumine, lysine, and salts thereof, and most preferably, the additive is selected from arginine, lysine, and salts thereof.

### (4) Preparation Comprising Additive, Nucleic Acid Molecule, and Collagen

The present invention provides a preparation comprising an additive of the present invention, a nucleic acid molecule and collagen, wherein the nucleic acid molecule is complexed with collagen to form a fine particle of controlled size.

The "nucleic acid molecule" used in the present invention can be any nucleic acid molecule as long as it has a property of polyanion around neutral pH region, including polynucleotides, oligonucleotides, DNA and RNA which are of natural- or modified type, from the viewpoint that a nucleic acid molecule forms a complex with collagen as a positively charged polycation around neutral pH region through the electrostatic interaction. In the case of DNA molecule, it may be a plasmid DNA, cDNA, genomic DNA, or a synthetic DNA. DNA and RNA can be a double- or single-stranded molecule. In the case of single-stranded molecule, it can be a coding or non-coding strand. Further, the "nucleic acid molecule" may be obtained synthetically or isolated from cells, and from the aspect of function, examples thereof include a plasmid DNA (may be referred to as "pDNA") as a gene, an RNA/DNA chimera oligonucleotide or a DNA oligonucleotide used in the gene conversion, an antisense DNA used in the regulation of gene expression, an oligonucleotide such as siRNA (small interfering RNA), a ribozyme that cleaves itself or other RNAs, a decoy oligonucleotide that is a double stranded DNA having the same sequence as the binding site of transcriptional factor, and the like. Modified nucleic acid molecule includes a DNA or an RNA derivative, wherein the derivative refers to a nucleic acid which has undergone chemical modifications at the phosphate, sugar or base portion of internucleotide to avoid enzymatic degradations. Examples include nucleic acid molecules having chemical modifications for the purpose of improving the stability to nuclease, specifically, those wherein one of oxygen atom of phosphodiester bond is replaced by a sulfur atom (phosphorothioate), a methyl group (methyl phosphonate) or an amino group (phosphoroamidate), and two oxygen atoms of phosphodiester bond are replaced by sulfur atoms (phosphorodithioate), or a sulfur atom and a methyl group (methylphosphorothioate). In addition, examples include those wherein the sugar moiety is modified, such as 2'-O-methyl RNA, 2'-O-methoxyethyl RNA, or Locked nucleic acid(trade-mark) (LNA), and the like. Further, nucleic acid molecules include viruses such as adenovirus, retrovirus, and the like.

The nucleic acid molecule is not limited to a particular chain length. In the case of oligonucleotide or ribozyme, the chain length is preferably from 5 mer to 100 mer, more preferably from 5 mer to 30 mer. In the case of oligonucleotide used as an antisense or siRNA, the chain length is about 20 mer, and in the case of RNA/DNA chimera oligonucleotide or a DNA oligonucleotide for gene conversion, the chain length is from dozens to a hundred base pairs. The plasmid DNA used as a nucleic acid of the present invention is not limited to a particular size and selected appropriately from those having a size which is suited to be prepared efficiently by genetic engineering procedures, and, when introduced into cells, can express effectively genetic information encoded thereby. Such a plasmid DNA is generally a polymer of several thousands to several tens thousands of base pairs.

The "nucleic acid molecule" used in the present invention is not limited to a particular sequence and therefore includes those which are commercially available or will be developed in the future. Examples of "nucleic acid molecule" include the following antisense DNAs which are currently used clinically.
Isis Pharmaceuticals:
Vitravene^{™}: 5'-GCG TTT GCT CTT CTT CTT GCG-3', SEQ ID NO: 1,
Affintak^{™}: 5'-GTT CTC GCT GGT GAG TTT CA-3', SEQ ID NO: 2,
Alicaforsen^{™}: ISIS2302 (5'-GCC CAA GCT GGC ATC CGT CA-3', SEQ ID NO: 3), ISIS2503 (5'-TCC GTC ATC GCT CCT CAG GG-3', SEQ ID NO: 4), ISIS 14803 (5'-GTG CTC ATG GTG CAC GGT CT-3', SEQ ID NO: 5), ISIS104838 (5'-GCT GAT TAG AGA GAG GTC CC-3', SEQ ID NO: 6).
GENTA Incorporated:
Genasense^{™}: 5'-TCT CCC AGC GTG CGC CAT-3', SEQ ID NO: 7, Hybridon Inc.:
GEM^{™} 231: 5'-GCG UGC CTC CTC ACU GGC-3', SEQ ID NO: 8, Epigenesis Pharmaceuticals Inc.:
EPI-2010: 5'-GAT GGA GGG CGG CAT GGC GGG-3', SEQ ID NO: 9

"Collagen" used in the present invention is the main protein in mammals, constituting about one third of the total protein content. As a basic structure, three collagen strands form triple helix, and there are at least 19 types of collagen molecule and 33 kinds of a chains. Among them, type I collagen is the main component of skin, bone and tendons, which has molecular weight of about 300,000 and is a rod-like molecule (length: about 300 nm; diameter: 1.5 nm) composed of two α1 polypeptide chains (Type I) and one α2 polypeptide chain (Type I) each polypeptide chain having a molecular weight of about 100,000. Collagens align regularly with a phase lag of 67 nm each from the adjacent molecules to form microfibrils which then assemble together to form water-insoluble fibrils (fibers). In the present invention, collagen is characterized in that it is in the monomolecular-dispersed state or fine fibrillar state, and that it disperses well in a solution state.

There are no particular limitations regarding origin or molecular species (type) of collagen, and any collagens including derivatives can be used as long as the fundamental physical properties as a collagen is maintained. It is preferred to use, in general, type I collagens derived from mammals such as bovine, porcine or human, birds, fishes (including cultured cells and genetically modified products). Further, it is possible to produce, as a genetically modified product, novel collagen molecules by selecting three α-chains arbitrarily, but it is more practical to prepare and use homo trimers composed of three α-chains of the same kind. Although collagen is a less antigenic protein by nature, atelocollagen is preferable collagen when a heterogeneous collagen is administered to human, because atelocollagen is almost free of problems related to antigenicity. That is, in atelocollagen, the telopeptide portions which contain the principal antigenic regions at the both termini of collagen molecule are enzymatically deleted.

Collagen derivatives usable include collagens with modified side chains or cross-linked collagens. Examples of collagens with modified side chains include methylated collagen, and examples of cross-linked collagens include those treated with glutaraldehyde, hexamethylene diisocyanate or epoxy compounds, and the like (Fragrance Journal, 1989-12, 104-109; JP-7-59522, B).

It was reported that nucleic acid molecules and collagens, when combined together, electrostatically and/or physically interact to form complexes, whereby the transfer of nucleic acid molecules into cells is facilitated and the gene expression can be sustained (Non-Patent document3).

As used herein, the term "a fine particulate complex of controlled size/complex in the form of a fine particle of controlled size" refers to a complex which has a size enabling the complex to maintain the dispersed state while preventing rapid sedimentation, and which can be administered through an ordinary injection needle without difficulty. Among ordinary injection needles with 30 to 16 gauge, specifically, the complex has a size adapted to pass through a 23 to 21 gauge (inside diameter ("ID"): about 0.4 - 0.6 mm) injection needle used for intra-venous or -arterial administration or intramuscular administration, more preferably a 27 to 24 gauge (ID: about 0.2 - 0.4 mm) injection needle used for intra- or sub-cutaneous administration. The term "controlled size" used for such a complex means that the complex is in the form of a particle having a size of not greater than 100 µm preferably not greater than 10 µm, wherein the term "size of not greater than 10 µm" means that the major axis is 10 µm or below when observed by microscope or that the particle can pass through a filter having a pore size of 10 µm (e.g., isopore filter, Millipore).

Accordingly, the expression "a/the nucleic acid molecule is complexed with collagen to form a fine particle(s) of controlled size" means that the complexes of a nucleic acid molecule and collagen are in the form of a particle of not greater than 100 µm, and free of aggregated complexes of greater than 100 µm, or the majority of complexes are in the form of a fine particle of not greater than 10 µm, specifically, at least 70%, preferably 80 % of the complexes are not greater than 10 µm, more preferably, at least 70% of the complexes of collagen and a nucleic acid in the preparation are not greater than 5 µm, and especially for intravascular administration, at least 80 % thereof are not greater than 5 µm.

The term "a complex which has a size enabling the complex to maintain the dispersed state while preventing rapid sedimentation" means that the complex has an appropriate size so that it can pass through an ordinary injection needle without difficulty on administration, and, in terms of the gauge of injection needle, it can pass injection needles of 23 to 21 gauge (internal diameter: about 0.4-0.6 mm), or the like. It is considered that majority of such complexes are not greater than about 10 µm.

Specifically, the preparation of the present invention is the one which comprises an additive comprising at least one substance selected from arginine, trometamol, meglumine, lysine, histidine, monoethanolamine, diethanolamine, triethanolamine, succinic acid, citric acid, tartaric acid, lactic acid and salts thereof, a nucleic acid molecule, and collagen, wherein the complex of the nucleic acid and collagen is in the form of a fine particle of controlled size, for example, at least 70% of fine particles are not greater than 10 µm in size. An additive selected from arginine, lysine, and salts thereof is particularly preferred.

One of preferred embodiments of the present invention is a liquid formulation wherein,
(1) the concentration of nucleic acid molecules is 0.001 - 100 mg/ml, preferably 0.001 - 50 mg/ml, more preferably 0.001 - 10 mg/ml,
(2) the concentration of collagens is 0.001 % - 10 %, more preferably 0.01 % - 2 %,
(3) the total concentration of additives is 1 - 10 %, and/or
(4) the pH of aqueous solution is 5-9, more preferably 6 - 8.

In another embodiment, the preparation of the present invention is the one which comprises an additive comprising at least one substance selected from arginine, trometamol, meglumine, lysine, triethanolamine, and salts thereof, a nucleic acid molecule and collagen, wherein the complex of the nucleic acid and collagen is in the form of a fine particle of controlled size, for example, at least 70% of fine particles are not greater than10 µm in size. An additive selected from arginine, lysine, and salts thereof is particularly preferred.

Examples of this preparation include a liquid formulation and a dry formulation. The drying-method for preparing a dry formulation is not limited to any particular method as long as the stability of nucleic acid can be kept. Examples include freeze-drying (lyophilization) method, airdrying method and spray-drying method. As a dry formulation, a lyophilized formulation prepared by freeze-drying method is preferred.

In the liquid formulation of this embodiment, the fine particulate complexes of controlled size of a nucleic acid molecule and collagen are prevented from aggregating and well dispersed by the action of the above-mentioned organic base additive of the present invention. The dry formulation is also useful for preparing a liquid formulation by reconstitution wherein the prevention of aggregation and the good dispersibility of particles are maintained.

The term "the fine particles ... are prevented from aggregating and well dispersed" means that the particles are prevented from aggregating and maintain the fine-particulate state of 10 µm or below, and free from aggregates larger than 100 µm under non-refrigeration conditions around a temperature ranging from room temperature to body temperature for 3 hours or longer and at least for 12 hours.

The particular usefulness of lyophilized formulation resides in that, when reconstituted (rewatered), the dispersibility is maintained without causing aggregation.

The preparation of the present invention can include a pharmaceutically acceptable additive(s) in addition to the fine particulate complex of a nucleic acid molecule and collagen, if needed. Examples of pharmaceutically acceptable additives include isotonizing agents, pH adjustors, soothing agents, etc. when the fine particulate complex is used as an injection; and excipients, disintegrating agents, etc., when the fine particulate complex is used as a solid preparation, which are described in Japanese Handbook of Pharmaceutical Excipients (Japan Pharmaceutical Excipients Council). Specific examples include salts for keeping the pH at 6 - 8 and saccharides for making isotonic with cells to be loaded.

An effect arisen from the fact that a complex of nucleic acid molecule with collagen has a controlled size is not only influences of the size of particle of complex on the biodistribution and medical effects after administration, but also critical significances in the quality control required for pharmaceutical agents, because that fact makes it possible to prevent aggregation and improve uniformity when preparing or using a preparation. In the formulation of the present invention, the particulate complexes show sustainable dispersibility and can be maintained stably without aggregation when they are left at room temperature or body temperature for about 3 hours or longer. This feature eliminates the necessity of rigid temperature regulation from the time of production to administration of the formulation, and makes its handling easy. The elimination of rigid temperature regulation during the manufacturing processes is of great advantage in the industrialization. On the other hand, a preparation of a complex of a nucleic acid molecule and collagen to which the present invention is not applied has drawbacks. For example, large aggregates are often formed immediately after temperature rise and the preparation becomes clouded, which causes a significant change of properties and also makes it difficult to administer by injection or to ensure the uniformity during the manufacturing process.

Another effect arisen from the fact that a complex of nucleic acid molecule and collagen has a controlled size is that it is possible to obtain complexes adapted to further processing for formulating For example, when a complex is to be included in other pharmaceutical carrier especially a fine particle used in the DDS such as microcapsule or microsphere, nanocapsule or nanosphere, liposome, emulsion and the like, said complex must be in the form of uniform particle having a size smaller than the fine particle encapsulating the same. However, conventional complexes are not uniform and have a size larger than fine particles for encapsulation, and therefore could not be included therein. In the case of the fine particulate complexes of the present invention, for example, when Arg is used as an additive, most of complexes obtained have a size small enough to pass through a 400 nm filter, and hence can be included in fine particles. For the production, a conventional formulation process can be applied by using complexes of the present invention in the state of aqueous solution or dry form.

### (5) Preparation for Transferring (Transporting) Nucleic Acid Molecule into Desired Cells

The preparation of the present invention can be used for transferring (transporting) a nucleic acid molecule into a desired cell. When the present preparation is in the solid form, it is loaded onto cells after converting into a solution using purified water, physiological saline, or a buffer isotonic with a living body.

Examples of desired cells include animal cells used in ordinary experiments, cells, tissues or organs which serve as the target of gene therapy. A nucleic acid molecule is hardly transferred into cells by itself when administered to cells in vitro in the presence of serum; however, a nucleic acid can be transferred into cells efficiently for the first time after forming fine particulate complexes with collagen. Thus, the preparation of the present invention is useful not only in a screening method or as a reagent therefor, but also in gene therapy as a preparation.

When the nucleic acid molecule is a plasmid DNA or a vector such as virus used in gene therapy, it is preferably constructed in a form suited to express genetic information encoded thereby intracellularly when transferred into cells, specifically, in the form of vectors comprising elements necessary for expression of intended gene such as promoter, or for enabling integration into chromosomes.

It is essential to use a method with the least influence on cells possible, when introducing a plasmid DNA and antisense DNA, siRNA, or the like into cells. However, it is considered that most of conventional methods for introduction would be highly cytotoxic. On the contrary, the preparation of the present invention uses a complex with collagen which is inherently present in a living body and contacts with cells, and therefore hardly does damage to cells. This makes it possible to measure the function of a nucleic acid molecule transferred without noise.

In a particular method of use of the present preparation, a preparation of the present invention comprising fine particulate complex of collagen with a plasmid DNA, an adenovirus vector expressing a gene of which function is to be elucidated; an antisense oligonucleotide suppressing the expression of a gene of which function is to be elucidated; or a siRNA; is coated and arranged on the solid surface of the culture plate. Solid plates as used herein include 96-well multiwellplates and microplates. After the coated complex particles are dried and immobilized on the solid, cells are seeded and cultured on the plate for several days. The coated complex-particles (complexes in the form of particle, particulate complexes) are transferred efficiently into cells attached to the coated part, and cause the expression of a gene whose function is to be examined, or inhibit the expression of the same for a long period of time. After a few days, the function of target gene can be clarified by examining the morphology of the cells, the level of the gene expression in the cells, or the kinds or the amount of proteins produced by the cells.

### Examples

The present invention is further illustrated by the following Examples, but is not restricted by these Examples in any way.

### Example 1

### Microparticulation Effect of Arginine on Complexes of Oligonucleotide and Collagen

Complexes of collagen and antisense DNA were prepared by using arginine hydrochloride as an additive, a phosphorothioate antisense DNA having a sequence (5'-TGCATCCCCCAGGCCACCAT-3', SEQ ID NO: 10) against a cell adhesion molecule mouse ICAM-1 as an oligonucleotide, and atelocollagen as a collagen (2% atelocollagen solution). They were mixed in PBS (pH 7.4) to the final concentration of 2% arginine, 0.1mg/ml antisense DNA, and 0.05 % collagen, and the mixture was allowed to stand overnight in a refrigerator (4°C - 10°C) to obtain complexes of collagen and antisense DNA.

As a Comparative Example, complexes were prepared by mixing oligonucleotide and atelocollagen in a similar manner to the above except that arginine is not included.

In the same manner, complexes of the present invention and another Comparative Example were prepared using a phosphorothioate antisense DNA having a sequence (5'-AACCCATCGGCTGGCACCAC-3', (SEQ ID NO: 11) which is as an antisense DNA against an inflammatory cytokine TNF-α, with and without 2% arginine, respectively.

The resultant complexes were filtered through the isopore filter (pore size: 10 µm, Millipore) to examine the size, and the amount of atelocollagen in the filtrate was measured. The results are shown in the following Table 1.

**Table 1**

| Sample No. | | Kind of additive Conc. | Kind of oligonucleotide Conc. | Atelocollagen Conc. | Solvent | Visual check | Percent of Atelocollagen passed through 10 µm pore filter |
|---|---|---|---|---|---|---|---|
| Ex.* | 1-1 | arginine, 2% | antisense ICAM-1 0.1mg/ml | 0.05% | PBS | clear | 88% |
| CEx. ** | 1-2 | -- | antisense ICAM-1 0.1mg/ml | 0.05% | PBS | slightly white turbidity | 36% |
| Ex. | 1-3 | arginine, 2% | antisense TNF-α 0.1mg/ml | 0.05% | PBS | clear | 88% |
| CEx. | 1-4 | -- | antisense TNF-α 0.1mg/ml | 0.05% | PBS | slightly white turbidity | 27% |

| | | | | | | | |
|---|---|---|---|---|---|---|---|
| Ex.*: Example CEx*: Comparative Example | | | | | | | |

As shown in Table 1, samples 1-1 and 1-3 prepared as Examples gave clear solution, and about 90% of atelocollagen passed through a filter (pore size: 10 µm). On the other hand, samples 1-2 and 1-4 prepared as Comparative Examples became slightly white-turbid and only about 30% of atelocollagen passed through a filter (pore size: 10 µm).

Under the conditions of the Example 1, the molar concentration of antisense DNA used is about 10 times as high as that of atelocollagen, and hence the excess antisense DNA may be present in the free form; however, all the atelocollagen are considered to form complexes with antisense DNA. Accordingly, the results indicate that, in the case of complexes of the present invention, about 90 % thereof are in the form fine particles of not greater than 10 µm on the basis of the amount of atelocollagen; while in the case of Comparative Example, about 70 % thereof are present as huge aggregates of greater than 10 µm.

### Example 2

### Microparticulation Effect of Various Additives on Complexes of Oligonucleotide and Collagen

Complexes were prepared by mixing a phosphorothioate antisense DNA against mouse ICAM-1 and atelocollagen in a neutral aqueous solution using an additive of the present invention, said additive being in the form of a hydrochloride or having been neutralized with various acids, and allowing the mixture to stand overnight in a refrigerator (4°C - 10°C). The kinds of additives used and the final concentration of respective ingredients are summarized in Table 2.

To the respective samples was added single-stranded nucleic acid fluorescence staining regent YOYO (Molecular Probes) in order to stain the antisense DNA, and the samples were observed by fluorescent microscopy. Particles of greater than several µm can be discriminated by fluorescent microscopy. Every Examples gave uniform staining profile, and no particles exceeding 10 µm were observed.

**Table 2-1**

| Sample No. | | Kind of additive Conc. | Salt of additive or acid for neutralization | Kind of oligonucleotide Conc. | Atelo-collagen Conc. | Solvent | Observation by fluorescent microscopy |
|---|---|---|---|---|---|---|---|
| Ex. | 2-1 | arginine 4% | HCl | antisense ICAM-1 0.25mg/ml | 0.1% | 0.1M PB* | no particles with the major axis over 10 µm |
| Ex. | 2-2 | arginine 2% | HCl | antisense ICAM-1 0.25mg/ml | 0.1% | 0.1M PB | no particles with the major axis over 10 µm |
| Ex. | 2-3 | arginine 2% | HCl | antisense ICAM-1 0.5mg/ml | 0.3% | 0.1M PB | no particles with the major axis over 10 µm |
| Ex. | 2-4 | arginine 4% | HCl | antisense ICAM-1 0.25mg/ml | 0.02% | 0.01M PB | no particles with the major axis over 10 µm |
| Ex. | 2-5 | arginine 4% | HCl | antisense ICAM-1 0.25mg/ml | 0.3% | 0.01M PB | no particles with the major axis over 10 µm |
| Ex. | 2-6 | arginine 4% | HCl | antisense ICAM-1 0.1mg/ml | 0.05% | water | no particles with the major axis over 10 µm |
| Ex. | 2-7 | arginine 4% | HCl | antisense ICAM-1 0.25mg/ml | 0.1% | water | no particles with the major axis over 10 µm |
| Ex. | 2-8 | arginine 4% | HCl | antisense ICAM-1 1mg/ml | 1% | water | no particles with the major axis over 10 µm |
| Ex. | 2-9 | arginine 8% | HCl | antisense ICAM-1 1mg/ml | 1% | water | no particles with the major axis over 10 µm |
| Ex. | 2-10 | arginine 4% | citric acid (1.4%) | antisense ICAM-1 0.25mg/ml | 0.1% | water | no particles with the major axis over 10 µm |
| Ex. | 2-11 | arginine 4% | tartaric acid (1.7%) | antisense ICAM-1 0.25mg/ml | 0.1% | water | no particles with the major axis over 10 µm |

| | | | | | | | |
|---|---|---|---|---|---|---|---|
| *PB: phosphate buffer | | | | | | | |

**Table 2-2**

| Sample No. | | Kind of additive Conc. | Salt of or additive or acid for neutralization | Kind of oligonucleotide Conc. | Atelocollagen Conc. | Solv. | observation by fluorescent microscopy |
|---|---|---|---|---|---|---|---|
| Ex. | 2-12 | arginine 4% | succinic acid (1.3%) | antisense ICAM-1 0.25mg/ml | 0.1% | water | no particles with the major axis over 10 µm |
| Ex. | 2-13 | trometamol 5% | HCl | antisense ICAM-1 0.25mg/ml | 0.1% | water | no particles with the major axis over 10 µm |
| Ex. | 2-14 | trometamol 5% | citric acid (2.4%) | antisense ICAM-1 0.25mg/ml | 0.1% | water | no particles with the major axis over 10 µm |
| Ex. | 2-15 | trometamol 5% | succinic acid (2.1%) | antisense ICAM-1 0.25mg/ml | 0.1% | water | no particles with the major axis over 10 µm |
| Ex. | 2-16 | meglumine 6% | HCl | antisense ICAM-1 0.25mg/ml | 0.1% | water | no particles with the major axis over 10 µm |
| Ex. | 2-17 | meglumine 6% | citric acid (1.9%) | antisense ICAM-1 0.25mg/ml | 0.1% | water | no particles with the major axis over 10 µm |
| Ex. | 2-18 | histidine 1.5% | HCl | antisense ICAM-1 0.25mg/ml | 0.1% | water | no particles with the major axis over 10 µm |
| Ex. | 2-19 | lysine 3% | HCl | antisense ICAM-1 0.25mg/ml | 0.1% | water | no particles with the major axis over 10 µm |
| Ex. | 2-20 | monoethanol- amine 6% | HCl | antisense ICAM-1 0.25mg/ml | 0.1% | water | no particles with the major axis over 10 µm |
| Ex. | 2-21 | diethanol- amine 6.1% | HCl | antisense ICAM-1 0.25mg/ml | 0.1% | water | no particles with the major axis over 10 µm |
| Ex. | 2-22 | triethanol- amine 6.7% | HCl | antisense ICAM-1 0.25mg/ml | 0.1% | water | no particles with the major axis over 10 µm |

### Example 3

### Inhibitory Effect of Various Additives on Aggregation of Fine Particulate Complexes in Relation to Temperature

As an additive, arginine or trometamol was used, wherein the former was used in the form of hydrochloride and the latter was used after neutralization with hydrochloric acid. Complexes were prepared by mixing the additive, a phosphorothioate antisense DNA against mouse ICAM-1 as an oligonucleotide, and atelocollagen in neutral 0.1M phosphate buffer (pH 6.5 - 7.9) to the final concentration of 2%, 0.125 mg/ml, and 0.3 %, respectively, and allowing the mixture to stand in a refrigerator (4°C - 10°C) for two days. As Comparative Examples, complexes were prepared in the same manner except that additive is not included, or urea (denaturant and solubilizing agent of protein), glycerin (glycerol, typical fiber disassembly agent of collagen disclosed in the Patent document 4), acetyl tryptophan Na (stabilizing agent of protein), or Tween 80 (surfactant) is selected and used. The final concentration of respective ingredients in the Examples and Comparative Examples are as shown in Table 3. To examine the aggregation inhibitory effect at room temperature, respective complexes were allowed to stand at room temperature for 18 hours and the state of particles before and after standing were compared. The results of visual check (observation) after standing are shown in Table 3. The fluorescence micrographs before and after standing are shown in Fig. 1.

Figure 1 shows that under the refrigeration condition before standing at room temperature, huge complexes over 100 µm were formed in the sample 3-3 (Comparative Example), while in the case where arginine (Examples 3-1), trometamol (Examples 3-2), urea (Comparative Example 3-4), or acetyl tryptophan Na (Comparative Example 3-7) was used, hardly any huge aggregates were formed, demonstrating the existence of microparticulation effect. However, in the cases of urea and acetyl tryptophan Na, aggregation of particles was observed after standing at room temperature. Further, in the case of glycerin (Comparative Example 3-5), aggregation (>10 µm) was observed after mixing collagen with DNA, which became greater after standing at room temperature. Tween 80 (Comparative Example 3-7) has no effect either, and aggregation of particles showed tendency to further progress after standing at room temperature to become huge.

On the other hand, in the sample where arginine (Example 3-1) or trometamol (Example 3-2) is included, fine and uniform complexes were maintained after standing at room temperature, indicating that they exert aggregation inhibitory effect even at room temperature.

**Table 3**

| Sample No. | | Kind of additiveConc. | Salt of additive or acid for neutralization | Kind of oligonucleotide Conc. | Atelocollagen Conc. | Solvent | After 18-hour-standing at room temperature (Visual observation) |
|---|---|---|---|---|---|---|---|
| Ex. | 3-1 | arginine 2% | HCl | antisense ICAM-1 0.125mg/ml | 0.3% | 0.1M PB | clear |
| Ex. | 3-2 | trometa mol (tris) 2% | HCl | antisense ICAM-1 0.125mg/ml | 0.3% | 0.1M PB | clear |
| CEx. | 3-3 | - | | antisense ICAM-1 0.125mg/ml | 0.3% | 0.1M PB | white turbidity |
| CEx. | 3-4 | urea 2% 0.125mg/ml | | antisense ICAM-1 | 0.3% | 0.1M PB | white turbidity |
| CEx. | 3-5 | glycerin 2% | | antisense ICAM-1 0.125mg/ml | 0.3% | 0.1M PB | white turbidity |
| CEx. | 3-6 | acetyl tryptoph an Na 1% | | antisense ICAM-1 0.125mg/ml | 0.3% | 0.1M PB | white turbidity |
| CEx. | 3-7 | Tween 80 0.1% | | antisense ICAM-1 0.125mg/ml | 0.3% | 0.1M PB | white turbidity |

### Example 4

### Effect of Additive on Freeze-Thawing

To confirm the effect of an additive of the present invention on the state of particulate complexes at the time of freeze-thawing, sample 2-6 (Example) and sample 1-2 (Comparative Example) were freezed at -20 °C. After confirming that the samples are completely frozen, thawing was conducted at room temperature. After thawing, samples were subjected to cold storage at 5 °C before returning to room temperature, and the particle size was then examined. This procedure was repeated twice. As a result, in the case of sample 2-6 (Example), no complexes over 10 µm were found in the fluorescence micrographs before and after freeze-thawing. When filtered through a filter (pore size: 0.4 µm), more than 90 % of collagens passed through. It was revealed that the complexes were maintained in the fine particulate state even after freeze-thawing. On the other hand, in the case of sample 1-2 (Comparative Example), huge aggregates over 100 µm were found before and after freeze-thawing. When filtered through a filter (pore sized: 10 µm), about 20 % of collagens passed through the filter before freezing; the percentage showed tendency to rather decrease after freeze-thawing, and 10-17 % collagens passed through the filter, indicating that aggregation of complex particles is possibly facilitated by freeze-thawing.

**Table 4**

| Sample No. | | State of Particles before freezing | | State of Particles after freeze-thawing | |
|---|---|---|---|---|---|
| | | fluorescence micrograph | filtration | fluorescence micrograph | filtration |
| Ex. | 2-6 | no aggregates (> 10 µm) were found | 90% or more collagen passed through ϕ0.4µm | no aggregates (> 10 µm) were found | 90% or more collagen passed through ϕ0.4µm |
| CEx. | 1-2 | aggregates (>100µm) were found | 20% collagen passed through ϕ10µm | aggregates (>100µm) were found | 10-17% collagen passed through ϕ10µm |

### Example 5

### Effect of Additive on Lyophilization

To examine the effect of an additive on the state of particle at the time of lyophilization, complexes each having the composition shown in Table 5-1 were prepared by a similar method to Example 1. In Examples 5-2, 5-3, a saccharide was added as an excipient.

After preparing the respective complexes, evaluation was carried out by observing the state of complex-particles before and after lyophilization using fluorescent microscopy. The lyophilized formulation of every Example, when reconstituted by adding water, dissolved rapidly to give clear aqueous solution. The results of microscopic observation are shown in Table 5-2.

**Table 5-1**

| Sample No. | | Kind of additive Conc. | Salt or acid for neutralization | Saccharide | Antisense ICAM-1 | Atelocollagen | Solv. |
|---|---|---|---|---|---|---|---|
| Ex. | 5-1 | arginine 4% | HCl | - | 0.25 mg/ml | 0.1% | water |
| Ex. | 5-2 | arginine 4% | HCl | sucrose 2% | 0.25 mg/ml | 0.1% | water |
| Ex. | 5-3 | arginine 4% | HCl | trehalose 2% | 0.25 mg/ml | 0.1% | water |

**Table 5-2**

| Sample No. | | Fluorescence micrograph before lyophilization | Fluorescence micrograph after lyophilization |
|---|---|---|---|
| Ex. | 5-1 | no aggregates (> 10 µm) were found | no aggregates (> 10 µm) were found |
| Ex. | 5-2 | no aggregates (> 10 µm) were found | no aggregates (> 10 µm) were found |
| Ex. | 5-3 | no aggregates (> 10 µm) were found | no aggregates (> 10 µm) were found |

### Example 6

### Microparticulation Effect on Complexes between Plasmid DNA and Collagen, and Inhibitory Effect on Aggregation by Temperature and Freeze-thawing Treatments

Complexes of plasmid DNA with collagen were prepared in the same manner as Example 1 using plasmid DNA (pCMV-LacZ, 4079 kb, molecular weight: about 2,500,000) in place of antisense DNA. The compositions are shown in Table 6-1. Respective complexes were allowed to stand at 5 °C or room temperature for one day, or freezed at -40 °C (one day) then thawed at room temperature. The particle state of complexes was observed by fluorescent microscopy. The fluorescent staining of plasmid DNA was conducted using picoGreen dsDNA Quantitation Reagent (Molecular Probes). The results are shown in Table 6-2 and Figure 2.

**Table 6-1**

| Sample No. | | Kind of additive Conc. | Salt or acid for neutralization | pDNA | Atelocollagen | Solv. |
|---|---|---|---|---|---|---|
| Ex. | 6-1 | arginine | HCl | 0.1 mg/ml | 0.05% | PBS |
| CEx. | 6-2 | - | HCl | 0.1 mg/ml | 0.05% | PBS |

**Table 6-2**

| Sample No. | | After 1-day-standing at 5 °C | After 1-day-standing at room temperature | After one time freeze-thawing |
|---|---|---|---|---|
| Ex. | 6-1 | no particles (>10µm) were found | no particles (>10µm) were found | no particles (>10µm) were found |
| CEx. | 6-2 | particles (>10µm) were found | aggregates found were found | aggregates were (>100µm) found |

It was revealed that, in the formation of complexes of plasmid DNA with collagen, complex particles over 10 µm and their aggregates were observed in Comparative Example sample 6-2. However, aggregation of fine particles was prevented in Example sample 6-1, and well dispersed complexes were obtained. Further, in the Comparative Example, aggregation showed tendency to be progressed by one-day-standing at room temperature or freeze-thawing treatments. In the case of Example, no aggregation was observed by these treatments, showing the effect of maintaining fine particle state.

### Example 7

### Inhibitory Effect of Various Additives on Aggregation of Complexes

Complexes each having the composition shown in Table 7 were prepared by a similar method to Example 3. As an additive, histidine (neutralized with hydrochloric acid), tartaric acid (neutralized with NaOH) or citric acid (Na salt) were used, and complexes were prepared by mixing an additive, a phosphorothioate antisense DNA against mouse ICAM-1 as an oligonucleotide and atelocollagen in neutral 0.1M phosphate buffer (pH 6.5 - 7.9) to the final concentration of 2%, 0.125 mg/ml, and 0.3 %, respectively, and allowing the mixture to stand in a refrigerator (4°C - 10°C) for two days. As Comparative Examples, complexes were prepared in the same manner without using an additive, or adding propylene glycol (typical fiber disassembly agent of collagen disclosed in the Patent document 4). The state of complexes were evaluated by visual check and observation by fluorescent microscopy. The results of visual check are shown in Table 7 and fluorescence micrographs are shown in Fig. 3

**Table 7**

| Sample No. | | Kind of additive Conc. | Salt of additive, acid or base for neutralization | Kind of oligonucleotide Conc. | Collagen Conc. | Solv. | Visual check |
|---|---|---|---|---|---|---|---|
| Ex. | 7-1 | histidine 1.5% | HCl | antisense ICAM-1 0.125mg/ml | 0.3% | 0.1M PB | clear |
| Ex. | 7-2 | tartaric acid 2% | NaOH | antisense ICAM-1 0.125mg/ml | 0.3% | 0.1M PB | clear |
| Ex. | 7-3 | citric acid 2% | NaOH | antisense ICAM-1 0.125mg/ml | 0.3% | 0.1M PB | clear |
| CEx. | 7-4 | - | | antisense ICAM-1 0.125mg/ml | 0.3% | 0.1M PB | white turbidity |
| CEx. | 7-5 | propylene glycol 2% | | antisense ICAM-1 0.125mg/ml | 0.3% | 0.1M PB | white turbidity |

The results of visual check showed that samples 7-1 to 7-3 (Examples) were all clear, while samples 7-4 and 7-5 (Comparative Examples) were white turbidity and nonuniform.

Microscopic observation (Fig. 3) showed that huge complexes over 100 µm were formed in the sample 7-4 (Comparative Example) which does not contain an additive. Similarly, in the case of propylene glycol (Comparative Example 7-5), which is a typical fiber disassembly agent of collagen disclosed in the Patent document 4, many huge complexes over 100 µm were observed after mixing collagen and DNA. Thus, microparticulation effect on complexes of collagen and DNA was not recognized. Contrary to the above, in the cases of histidine (Example 7-1), tartaric acid (Example 7-2) and citric acid (Example 7-3) of the present invention, huge complexes were hardly observed and microparticulation effect was recognized.

### Example 8

### Microparticulation Effect of Arginine on Complexes of Oligonucleotide of High Concentration and Collagen

A preparation was prepared using an additive and the like as shown in Table 8 by a method similar to Example 2 under the conditions below, and examined whether or not aggregates exist by fluorescent microscopy. The result of microscopic observation is shown in Table 8 and Fig. 4. The micrograph showed uniform staining profile, and there were no huge aggregates over 10 µm.

**Table 8**

| Sample No. | Kind of additive Conc. | Acid for neutralization | Kind of oligonucleotide Conc. | Atelocollagen Conc. | Solv. | fluorescence micrograph |
|---|---|---|---|---|---|---|
| Ex.8 | arginine 4% | HCl | antisense ICAM-1 40 mg/ml | 0.05% | water | no aggregates (>10µm) were found |

### Test Example 1

### Effect in Mouse Model of Dermatitis

To evaluate the medicinal effect of a preparation of the present invention, a preparation comprising antisense ICAM-1 as a nucleic acid molecule (Table 9) was prepared by a method similar to Example 2, and administered to mouse models of dermatitis (see, the evaluation system described in Hum Gene Ther. 2004 Mar;15 (3):263). The antiinflammatory effect was then evaluated.

**Table 9**

| Sample No. | | Kind of additive Conc. | Acid for neutralization | Kind of oligonucleotide Conc. | Atelocollagen Conc. | Solv. |
|---|---|---|---|---|---|---|
| Ex. | 9-1 | arginine 4% | HCl | antisense ICAM-1 0.1mg/ml | 0.1% | water |
| Ex. | 9-2 | 4% | HCl | antisense ICAM-1 0.1mg/ml | 0.05% | water |
| CEx. (Control) | 9-3 | - | - | antisense ICAM-1 0.1mg/ml | - | PBS |
| CEx. (Control) | 9-4 | arginine 4% | HCl | - | 0.05% | water |

The evaluation of medicinal effect on mouse model of dermatitis was conducted according to the following method.

Abdominal part of a mouse (aged 8 weeks, BALB/c Cr Slc, ♂) was shaved by electric clippers and razor. To the shaved part was applied 0.5 % 2,4-dinitrofluorobenzene (DNFB; solvent, acetone: olive oil = 4:1) (25 µL) 2 days consecutively for sensitizing the mouse. Six days after the second sensitization, the thickness of right auricle was measured under anesthesia using thickness gauge (Peacock) to obtain the former value. Then, 0.2% DNFB (15 µL) was applied to the right auricle to induce dermatitis, and 15 minutes later, the preparation (Example 9-1 or 9-2) was administered through caudal vein at the dose of 10 mL/kg in terms of antisense ICAM-1. Control groups tested include a group which received physiological saline, a group which received antisense ICAM-1 solution without atelocollagen (Comparative Example 9-3) and a group which received atelocollagen solution without antisense ICAM-1 (Comparative Example 9-4). After 24 hours from DNFB application, the thickness of right auricle was measured again, and the difference between the values before and after the induction of dermatitis was calculated to obtain Ear swelling index (× 10⁻³ mm). The mean values (±S.E.) of respective groups (n=5) were calculated and shown in Fig. 5.

The evaluation of medicinal effect on mouse model of dermatitis revealed that single administration of antisense ICAM-1 (Comparative Example 9-3) or atelocollagen (Comparative Example 9-4) did not show significant inhibitory effect on ear swelling compared to the group received physiological saline. However, the group received the preparation of the present invention (Example 9-1, 9-2) showed significant inhibitory effect on ear swelling. These results demonstrate that the preparation of the present invention, as a fine particulate complex of a nucleic acid molecule and collagen, has higher medicinal effect compared to the sole administration of antisense ICAM-1.

### Test Example 2

To examine the medicinal effect of lyophilized formulation of the present invention, a lyophilized formulation comprising antisense ICAM-1 as a nucleic acid molecule (Table 10) was prepared by a method similar to Example 5, administered to mouse models of dermatitis, and tested the antiinflammatory effect in the same manner as Test Example 1.

**Table 10**

| Sample No. | | Kind of additive Conc. | Acid for neutralization | Kind of oligonucleotide Conc. | Atelocollagen Conc. | Solv. |
|---|---|---|---|---|---|---|
| Example (Lyophilized 10-1 formulation) | | arginine 4% | HCl | antisense ICAM-1 0.1mg/ml | 0.05% | water |

The evaluation of medicinal effect on mouse model of dermatitis was conducted according to the following method.

Abdominal part of a mouse (aged 8 weeks, BALB/c Cr Slc, ♂) was shaved by electric clippers and razor. To the shaved part was applied 0.5 % 2,4-dinitrofluorobenzene (DNFB; solvent, acetone: olive oil = 4:1) (100 µL) once for sensitizing the mouse. Six days after the sensitization, the thickness of the both of right and left auricles was measured under anesthesia using thickness gauge (Peacock) to obtain the former values. Then, 0.2% DNFB (20 µL) was applied to the right and left auricles to induce dermatitis, and 15 minutes later, the lyophilized formulation (Example 10-1) previously reconstituted by adding water was administered through caudal vein at the dose of 10 mL/kg in terms of antisense ICAM-1. A control group tested received physiological saline. After 24 hours from DNFB application, the thickness of the both auricles was measured again, and the difference between the values before and after the induction of dermatitis was calculated to obtain Ear swelling index (× 10⁻³ mm). The mean values (±S.E.) of respective groups (n=6) were calculated and shown in Fig. 6.

The evaluation of medicinal effect on mouse model of dermatitis revealed that the group received the lyophilized formulation of the present invention (Example 10-1) showed significant inhibitory effect on ear swelling compared to the group received physiological saline (control). These results demonstrate that the fine complex of the present invention is useful also as a lyophilized formulation.

### INDUSTRIAL APPLICABILITY

According to the present invention, it is possible to provide a preparation comprising a fine particle of complex essentially composed of a nucleic acid molecule and collagen, which is capable of maintaining uniform dispersibility, in other words, which is uniform and shows splendid dispersibility and is suited for practical use. Specifically, the present invention provides a preparation comprising a fine particulate complex of controlled size, by inhibiting the aggregation of collagens themselves in a neutral aqueous solution, and also prevent the further progress of aggregation among complexes through collagen and nucleic acid molecule to result in huge aggregates, in the complexation of collagen with a nucleic acid molecule, by conducting the formation of a complex between a nucleic acid molecule and collagen in the co-existence of a certain organic base or an acid as an additive for collagen. The preparation of the present invention not only is applicable to nucleic acid pharmaceutical products but also has a potential of application in the nucleic acid-related reagent, diagnostic product, and kits.

## Claims

1. An additive for making a complex of a nucleic acid molecule and collagen in the form of a fine particle of controlled size, which comprises at least one substance selected from arginine, trometamol, meglumine, lysine, histidine, monoethanolamine, diethanolamine, triethanolamine, succinic acid, citric acid, tartaric acid, lactic acid, and salts thereof.

2. The additive according to claim 1, which is used for preparing an aqueous solution of pH 5-9 comprising the additive at a concentration of at least 1%, said aqueous solution being capable of adjusting the size in such a way that at least 70% of particles are not greater than 10 µm as fine particles of controlled size.

3. An aqueous solution comprising an additive described in claim 1 above and collagen, which is for making a complex of a nucleic acid molecule and collagen in the form of a fine particle of controlled size.

4. An aqueous solution comprising an additive described in claim 1 and collagen, which is for preparing a fine particulate complex of controlled size of a nucleic acid molecule and collagen by adding the nucleic acid to the aqueous solution.

5. An aqueous solution comprising an additive described in claim 1 for reconstituting a dry formulation comprising a nucleic acid molecule and collagen into a liquid formulation, which is for making a complex of a nucleic acid molecule and collagen in the form of a fine particle of controlled size.

6. An aqueous solution of pH 5-9 comprising an additive described in claim 1 at a concentration of at least 1%, which is capable of adjusting the size in such a way that at least 70% of particles are not greater than 10 µm as fine particles of controlled size.

7. A preparation comprising an additive described in claim 1, a nucleic acid molecule and collagen, wherein the nucleic acid molecule is complexed with collagen to form fine particles of controlled size.

8. The preparation according to claim 7, wherein at least 70 % of fine particulate complexes of controlled size are not greater than 10 µm in size.

9. The preparation according to claim 7 or 8, which is a liquid formulation.

10. An additive comprising at least one substance selected from arginine, trometamol, meglumine, lysine, triethanolamine, and salts thereof, which is:
(1) for making a complex of a nucleic acid molecule and collagen in the form of a fine particle of controlled size,
(2) for preventing fine particles from aggregating and allowing fine particles to well disperse in a liquid formulation comprising fine particulate complexes of controlled size of a nucleic acid molecule and collagen, and/or
(3) for maintaining the prevention of aggregation of fine particles and the good dispersibility of the same in a liquid formulation reconstituted from a dry formulation, which liquid formulation comprises a fine particulate complex of controlled size of a nucleic acid molecule and collagen.

11. An aqueous solution comprising an additive described in claim 10 and atelocollagen, which is for making a complex of a nucleic acid molecule and collagen in the form of a fine particle of controlled size.

12. An aqueous solution comprising the additive described in claim 10 for reconstituting a dry formulation comprising a nucleic acid molecule and collagen into a liquid formulation, which is
(1) for making a complex of a nucleic acid molecule and collagen in the form of a fine particle of controlled size, and
(2) for preventing fine particles from aggregating and allowing fine particles to well disperse in a reconstituted liquid formulation comprising fine particulate complexes of controlled size of a nucleic acid molecule and collagen.

13. A preparation comprising an additive described in claim 10, a nucleic acid molecule and collagen, wherein the nucleic acid molecule is complexed with collagen to form a fine particle of controlled size.

14. The preparation according to claim 13, wherein at least 70% of fine particulate complexes of controlled size are not greater than 10 µm in size.

15. The preparation according to claim 13 or 14, which is a liquid formulation comprising the additive described in claim 10 and wherein the fine particulate complexes of controlled size of a nucleic acid molecule and collagen are prevented from aggregating and well dispersed.

16. The preparation according to claim 13 or 14, which is a dry formulation comprising the additive described in claim 10, and which gives a liquid formulation by reconstitution wherein the prevention of aggregation of fine particles and the good dispersibility of the same are maintained.

17. The preparation according to any one of claims 7 to 9 and 13 to 16 which is to transporting a nucleic acid molecule into a desired cell.

18. The additive according to any one of claims 1, 2 and 10, wherein the collagen is atelocollagen.

19. The aqueous solution according to any one of claims 3 to 6, 11 and 12, wherein the collagen is atelocollagen.

20. The preparation according to any one of claims 7 to 9 an 13 to 17, wherein the collagen is atelocollagen.


SEQUENCE LISTING

<110>  Dainippon Sumitomo Pharma Co., Ltd.

<120>  Fine Particulate Preparation Comprising Complex of Nucleic Acid Molecule
       and Collagen

<130>  N2862 EP S3

<140>  EP 06729793.7
<141>  2006-03-23

<150>  JP 2005-086318
<151>  2005-03-24

<160>  11

<170>  PatentIn version 3.2

<210>  1
<211>  21
<212>  DNA
<213>  Artificial

<220>
<223>  antisense oligomer

<400>  1
gcgtttgctc ttcttcttgc g                                                21

<210>  2
<211>  20
<212>  DNA
<213>  Artificial

<220>
<223>  antisense oligomer

<400>  2
gttctcgctg gtgagtttca                                                  20

<210>  3
<211>  20
<212>  DNA
<213>  Artificial

<220>
<223>  antisense oligomer

<400>  3
gcccaagctg gcatccgtca                                                  20

<210>  4
<211>  20
<212>  DNA
<213>  Artificial

<220>
<223>  antisense oligomer

<400>  4
tccgtcatcg ctcctcaggg                                                    20


<210>  5
<211>  20
<212>  DNA
<213>  Artificial

<220>
<223>  antisense oligomer

<400>  5
gtgctcatgg tgcacggtct                                                    20


<210>  6
<211>  20
<212>  DNA
<213>  Artificial

<220>
<223>  antisense oligomer

<400>  6
gctgattaga gagaggtccc                                                    20


<210>  7
<211>  18
<212>  DNA
<213>  Artificial

<220>
<223>  antisense oligomer

<400>  7
tctcccagcg tgcgccat                                                      18


<210>  8
<211>  16
<212>  DNA
<213>  Artificial

<220>
<223>  antisense oligomer

<400>  8
gcggcctcct cacggc                                                        16


<210>  9
<211>  21
<212>  DNA
<213>  Artificial

<220>
<223>  antisense oligomer

<400>  9
gatggagggc ggcatggcgg g                                                21


<210>  10
<211>  20
<212>  DNA
<213>  Artificial

<220>
<223>  phosphorothioate antisense oligomer

<400>  10
tgcatccccc aggccaccat                                                  20


<210>  11
<211>  20
<212>  DNA
<213>  Artificial

<220>
<223>  phosphorothioate antisense oligomer

<400>  11
aacccatcgg ctggcaccac                                                  20